# EUROPEAN PATENT SPECIFICATION

(11) **EP 2 120 630 B1**
(45) Date of publication and mention of the grant of the patent: **10.11.2010**
(21) Application number: 08701918.8
(22) Date of filing: 23.01.2008
(51) Int. Cl.: A45D 29/18, A45D 34/04, A45D 34/06, A45D 40/24, A61M 35/00, A45D 29/04

(54) **TREATMENT DEVICE AND METHOD OF USING THE SAME**
BEHANDLUNGSGERÄT UND ANWENDUNGSVERFAHREN DAFÜR
DISPOSITIF DE TRAITEMENT ET SON PROCÉDÉ D'UTILISATION

(30) Priority: 23.01.2007 GB 0701306
(43) Date of publication of application: 25.11.2009
(73) Proprietor: LRC PRODUCTS LIMITED, London EC4V 6BW (GB)
(72) Inventor: GEORGE, Angela, Manchester M41 7HA (GB); WARD, Jonathan, Manchester M41 7HA (GB)
(74) Representative: Bennett, Adrian Robert J.
(86) International application number: PCT/GB2008/000244
(87) International publication number: WO 2008/090347

(56) References cited:
- EP-A- 1 378 188
- EP-A- 1 528 000
- WO-A-03/020440
- WO-A-2006/090971
- FR-A- 1 174 544
- FR-A- 2 859 078
- US-A- 3 786 820
- US-A- 5 897 262

## Description

The present invention relates to a treatment device and method of using the same, and more particularly, but not exclusively, to a container assembly and method of using the assembly for the treatment of fingernail and toenail conditions.

Onychauxis is an abnormal thickening of the toenail, increasing from the nail base to the free edge of the nail. It may be accompanied by yellow or slight brown colour changes in the nail plate and the nail may also become ridged. Often only the nail of the hallux (commonly known in humans as the big toe) is affected, but the condition may affect other nails as well. The excessive growth makes nail cutting difficult and makes the affected nail cosmetically unattractive. Pressure on the thickened nail, for example from shoes, may also cause pain and discomfort.

Onychauxis occurs following damage to the nail matrix, for which there may have been one or more of several causes:
- A single major trauma from a heavy blow or severe stubbing or repeated minor trauma from shallow shoes or pressure from footwear.
- Dermatological conditions such as eczema or psoriasis
- Poor peripheral circulation
- Systemic conditions such as Darier's disease (keratosis follicularis)

Trauma to the nail matrix results in the excessive production of onychocytes and the nail becomes progressively thicker as it grows along the nail bed. The proximal nail fold becomes shortened and unable to exert pressure on newly formed cells in order to flatten them as in a normal nail plate. Evidence also suggests that there is a greater vascularity in the nail fold and nail matrix areas. The nail matrix also produces an epidermal type of keratin with increased thickness of the nail plate and results in a thicker but softer intermediate nail layer.

Onychogryphosis is a more substantial thickening of the nail and is usually accompanied by significant nail curvature. As with Onychauxis, it is usually the result of damage to the nail matrix, sometimes accompanied by a long-term fungal infection. It is also commonly seen in the hallux but may occur in other toes as well as the fingernails. The affected nail is usually discoloured (brownish), grooved and ridged, and grows more quickly on one side than on the other leading to curvature.

A wide variety of toenail treatments are currently available that claim to neutralise, cover up or prevent toenail discolouration caused by keratin debris build-up, thickening of the nail or staining by nitrocellulose-based varnishes. A full treatment regime for optimum effectiveness should incorporate the following:
- Debridement of thickened nail keratin
- Chemical removal of discoloured keratin and/or bleaching of discoloured keratin
- Nail protection and masking of discolouration with a varnish covering

No single prior art treatment device allows for all three of these stages to be completed.

WO 2006/090971A describes a container assembly comprising two containers.

It is an object of the present invention to provide a container assembly and method which allows a more convenient treatment of fingernail and toenail conditions.

A first aspect of the present invention provides a container assembly comprising first and second containers and a closure element common to the containers, wherein each container comprises an opening for permitting access to the interior thereof, and wherein the assembly further comprises means for selectively securing each container to the closure element independently of the other container so as to close the opening of the container so secured, the closure element comprising a first applicator associated with the first container and a second applicator associated with the second container, wherein each applicator extends through the opening of the associated container when said opening is closed by the closure element and wherein the container assembly has an elongate cylindrical shape, **characterised in that** the container assembly further comprises a planar abrasive area located at one end of the container assembly and arranged so that the longitudinal axis of the container assembly is at an angle to the abrasive area.

One of the two containers may be used to contain a composition for the chemical removal of discoloured keratin and/or bleaching of discoloured keratin. The other of the containers may contain a covering to protect a nail and possibly to also mask discolouration. It will be understood that, in use, a first container may be removed from the remainder of the container assembly. The contents of this container may then be applied to a nail with the first applicator. The second container may remain secured to the closure element and held by a user to allow the user to more readily manipulate the first applicator. Once the contents of the first container has been applied to a nail, the first container may be closed by again securing it to the closure element. The second container may then be removed from the closure element and the contents thereof applied to the nail with the second applicator. The first container may be held during this process so as to allow a more ready manipulation of the second applicator. Once the contents of the second container has been applied to the nail, the second container may be closed by again securing it to the closure element. The quantity of composition held in the first and second containers may be such that this process may be repeated several times on the same nail and/or on other nails.

Further features of the first aspect of the present invention are provided as recited in any of the appended dependent claims 2 to 9 and 15 to 24.

A second aspect of the present invention provides a method as recited in the appended claim 10.

Further features of the second aspect of the present invention are provided as recited in any of the appended dependent claims 11 to 14.

Embodiments of the present invention will now be described with reference to the following drawings, in which:
Figure 1 is a perspective view of a first embodiment of the present invention arranged in a closed configuration;
Figure 2 is a perspective view of the first embodiment arranged in an open configuration;
Figure 2a is an enlarged view of a portion of the first embodiment highlighted in Figure 2 with circle A;
Figure 3 is a side view of the first embodiment;
Figure 3a is an end view of the first embodiment;
Figure 4 is a front view of the first embodiment;
Figure 5 is a cross-sectional front view of the first embodiment;
Figure 6 is a side view of a first container of the first embodiment;
Figure 6a is an enlarged view of a portion of the first embodiment highlighted in Figure 6 with circle A;
Figure 7 is a side view of a second container of the first embodiment;
Figure 8 is a perspective view of a second embodiment of the present invention arranged in a closed configuration;
Figure 9 is a cross-sectional front view of the second embodiment; and
Figure 10 is a side view of a second container of the second embodiment.

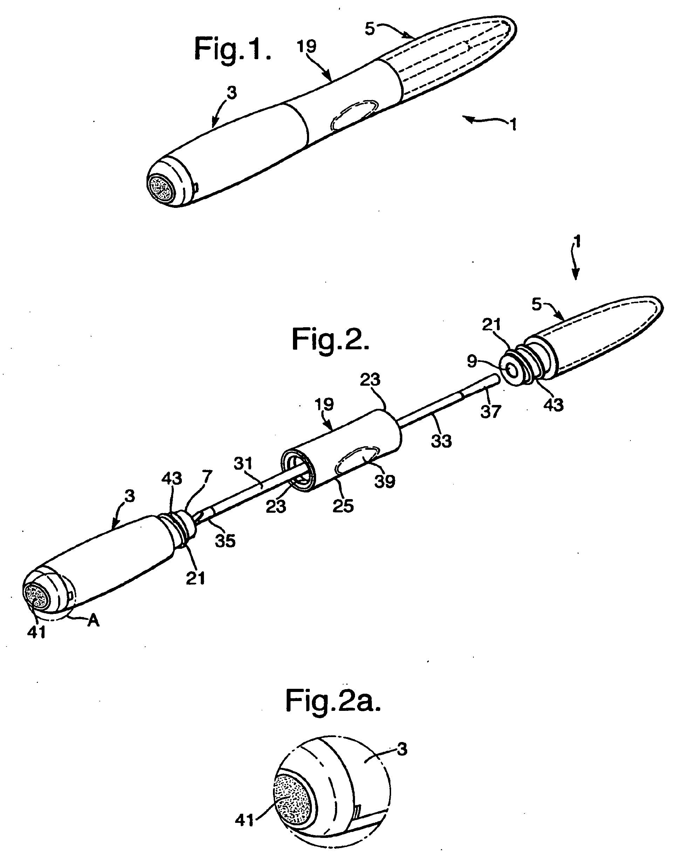

A first treatment device 1 according to the present invention is shown in Figures 1 to 7 of the accompanying drawings. The treatment device 1 comprises first and second containers 3, 5 which, in use, contain a substance or composition to be applied to a part of the body (for example, a fingernail or toenail) of the user.

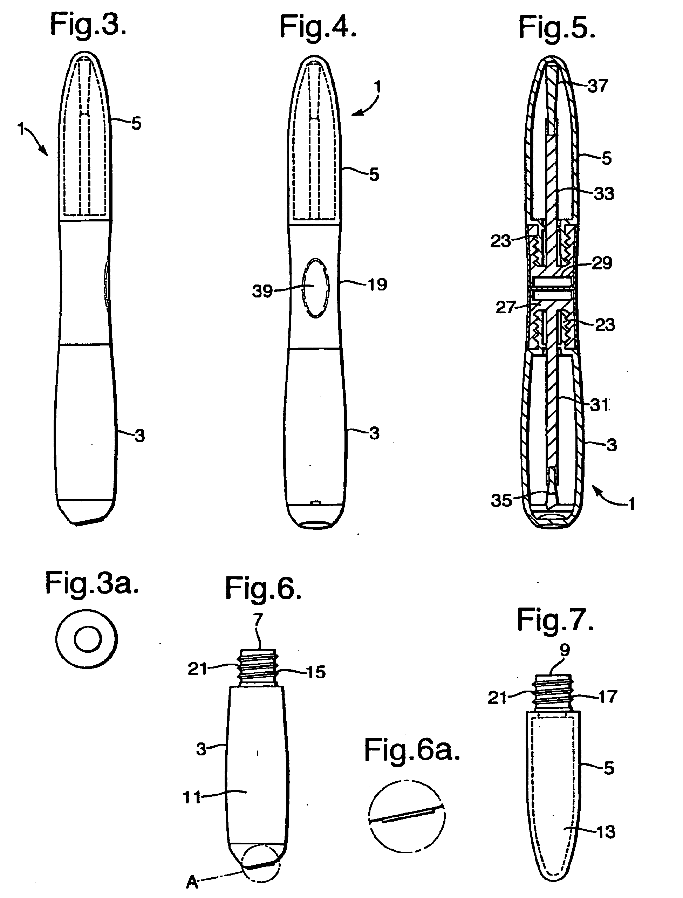

The containers 3, 5 have different shapes, but are similar in appearance in as much as they are both generally cylindrical and have a similar length and diameter. The shape of the two containers 3, 5 is most clearly illustrated in Figures 3, 4 and 5 of the accompanying drawings. It will be seen that each container 3, 5 tapers at one end so as to close said end, whereas an opposite end is provided with an opening 7, 9 by means of which access may be gained to the interior 11, 13 of the container 3, 5 (see Figures 6 and 7).

The opening 7, 9 of each container 3, 5 is surrounded by a cylindrically shaped neck portion 15, 17 having an outer diameter which is less than that of the remainder of the container 3, 5. The neck portion is provided for receiving a generally cylindrically shaped closure element 19 (described in greater detail below). The dimensions and contours of the container and closure element are such that, when the container is closed by the closure element, the two components abut one another without any discontinuity or step at the junction of their outer cylindrical surfaces. In other words, the outer surface of the closure element forms a smooth contour line with the outer surface of the associated container. This ensures the closure element and container combine to have a pleasant aesthetic appearance. The arrangement is also comfortable for a user when, for example, carried in a pocket against the skin, and reduces the risk of the container being inadvertently opened during general handling.

The exterior cylindrical surface of the neck portion 15, 17 of each container 3, 5 is provided with an external screw thread 21 for screw threadedly engaging with an internal screw thread 23 provided on a interior cylindrical surface of the closure element 19. As will be understood from the following description, the closure element 19 may be thereby securely fastened to each container 3, 5.

The closure element 19 comprises a body portion 25 having a generally cylindrical shape. An interior cylindrical surface at each end of the body portion 25 is provided with the abovementioned screw threads 23 so that the first container 3 may be screw threadedly secured to one end of the closure element 19 whilst the second container 5 is screw threadedly secured to the other end of the closure element 19.

A gasket (not shown) is provided about each neck portion 15, 17 so as to provide a seal between the body portion 25 and the associated container 3, 5. The provision of the gaskets assists in preventing the contents of the containers 3, 5 from leaking and from drying out during storage. Nevertheless, in order to allow excess internal pressure within the containers 3, 5 to be relieved, appropriate gas venting means is provided. This gas venting means takes the form of a slot or cut 43 in the external screw threads 21 extending along the length of the neck portion 15, 17. The slot extends longitudinally along the neck portion 15 (as shown in Figure 2), but alternatively may extend helically along the neck portion 15, 17.

The internal screw threads 23 provided on the body portion 25 may be provided with a similar venting means in addition to or as an alternative to the venting means of the external screw threads 21. It will be understood that the provision of a slot extending across the screw threads provides a small clearance gap through which gasses may pass under pressure, even when the containers 3, 5 are screw threadedly engaged with the closure element 19. A slot, groove or aperture may be provided in the neck portion 15, 17 and the body portion 25 so as to allow gas under pressure (i.e. in excess of the exterior atmospheric pressure) to pass from the interior of the respective container 3, 5 to the slot of the screw thread and so as to allow this gas to flow past the body portion 25 to the atmosphere.

First and second lateral walls 27, 29 extend across the interior of the closure element 19 and prevent fluid communication within the closure element 19 between the opposite ends thereof (see Figure 5). The lateral walls 27, 29 are positioned relative to the internal screw threads 23 so that, when each container 3, 5 is screwed into a closed position relative to the closure element 19, the walls 27, 29 abut the free end of a neck portion 15, 17 so as to sealingly close the associated container 3, S.

An elongate wand 31, 33 extends from the centre of each wall 27, 29. The position of each wand 31, 33 on the associated lateral wall 27, 29, and the length and cross-sectional size/shape of the wand, is such that each wand 31, 33 extends through the neck portion 15, 17 of its associated container 3, 5 so as to locate in the interior 11, 13 of said container 3, 5 when the container 3, 5 is closed by the closure element 19. Each wand 31, 33 may be manufactured integrally with the associated lateral wall 27, 29 and the remainder of the closure element 19 or may be a separate component connected to its associated lateral wall 27, 29 with appropriate means (for example, adhesive).

The end of each wand 31, 33 distal to the lateral walls 27, 29 is provided with an appropriate applicator. The applicator is secured to the wand with appropriate means (for example, adhesive) and is selected depending upon the type of composition/substance to be transported and applied by the applicator. The applicator provided for location in the first container 3 is a resiliently deformable wiper 35. The wiper 35 has a doe-foot shape. The applicator provided for location within the second container 5 is a brush 37. The combined length of the wand and applicator allows the applicator to be positioned at the end of the associated container distal to the container opening 7, 9.

Appropriate means may be provided for ensuring that a particular end of the body portion 25 is connectable with only one of the two containers 3, 5. For example, the neck portions 15, 17 may have different diameters and/or the pitch of the screw threads 21 on the containers 3, 5 may differ. In these examples, the ends of the body portion 25 of the closure element 19 will be adapted so as to be securable with its associated container 3, 5. For example, where different screw threads are provided on the containers 3, 5, the internal screw threads 23 on the body portion 25 will also be different so as to mate appropriately with their associated container 3, 5. It will be understood that screw threads of different pitches will not be threadedly engageable with one another and, accordingly, the connection of a container 3, 5 with the wrong end of the body portion 25 will be prevented.

Indicator means may also be provided to indicate to the user which container 3, 5 is to be received by a particular end of the closure body portion 25. For example, colour coding may be used wherein each end of the closure body portion 25 has a different colour which is the same colour as that provided on the associated container 3, 5.

The closure body portion 25 is provided with an area 39 for displaying a trade name relating to the device 1.

The treatment device 1 further comprises an abrasive area provided on an exterior surface thereof. The abrasive area is sufficiently coarse to readily debride nail keratin. The abrasive area is located at one end of the device as shown in the accompanying drawings.

With reference to Figures 2 and 2a in particular, it will be seen that the device 1 is provided with an abrasive area 41 positioned on the end of the first container 3 distal to the opening 7 of said container 3. The abrasive area 41 is planar and arranged so that the longitudinal axis of the device 1 is at an angle to the abrasive area 41 (rather than extending perpendicularly thereto). The angled arrangement of the abrasive area 41 at the end of the device 1 allows the ergonomic use of the abrasive area 41. For example, a user may hold the device 1 like a pen/pencil in one hand and conveniently rub the abrasive area 41 against the fingernails of the other hand.

It will be understood that an abrasive area 41 may be provided at both ends of the device 1.

The abrasive nature of said area 41 may be provided by moulding the surface of the treatment device 1 with a plurality of small raised elements. A roughened surface is thereby provided. Alternatively, the abrasive area 41 may be provided as an abrasive patch which is originally separate from the device 1 and secured to an external surface of the device 1 with suitable means (for example, adhesive). Suitable materials from which the abrasive patch may be manufactured include caborundum, etched metal, diamond etched metal and emery paper.

The containers 3, 5 and the closure element 19 (including the wands 31, 33) may be manufactured from any suitable plastics material such as PP, PE, ABS, SAN, PET, PETG. The applicator 35, 37 may be manufactured from a different material to that of the associated wand 31, 33. For example, the wiper 35 may be manufactured from TPE, POM or nylon. The containers 3, 5 may alternatively be manufactured from glass. This is particularly advantageous where it will be of interest or assistance to the user to be able to view the contents of the container 3, 5 whilst said container 3, 5 remains closed. For example, this will be the case where a container 3, 5 holds nail varnish.

The types of material used to manufacture the treatment device 1 will, in part, depend on the compositions/substances to be held by the containers 3, 5. For example, a glass container may be more suitable than a plastics container for holding a solvent-based varnish.

The first container 3 may contain either a keratolytic agent or a bleaching agent. Alternatively, the first container may contain both a keratolytic agent and a bleaching agent. The keratolytic agent will be understood to chemically break down and remove discoloured keratin whilst a bleaching agent acts to lighten the colour of discoloured keratin which remains. The keratolytic agent may be urea or an hydroxy-acid. The hydroxy-acid may be salicylic acid or lactic acid. The bleaching agent may be hydrogen peroxide.

The wiper applicator 35 associated with the first container 3 is particularly adapted for the transporting and applying the keratolytic agent and/or bleaching agent. Application may be assisted by providing the composition as a gel.

The second container 5 contains a covering varnish which may be solvent-based or water-based and may also contain an optical brightener.

In use of the treatment device 1, the abrasive area 41 is first used to physically remove thickened nail keratin. Treatment gel from the first container 3 is then applied with the wiper 35 to the nail undergoing treatment. Finally, covering varnish from the second container 5 is applied to the nail with the brush 37.

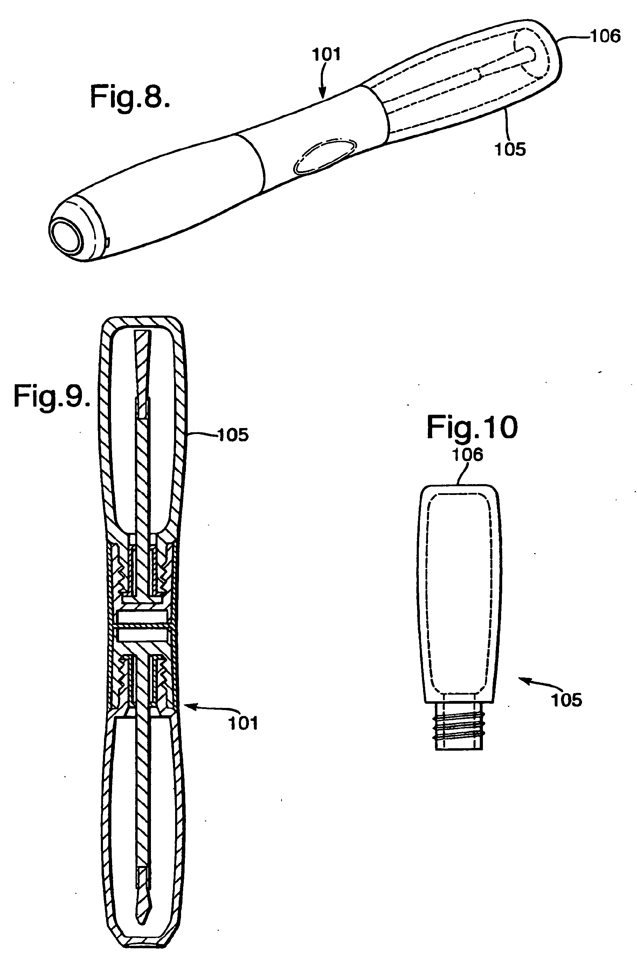

The present invention is not limited to specific embodiment described above. Alternative arrangements and suitable materials and compositions will be apparent to a reader skilled in the art. For example, a modified treatment device 101 is shown in Figures 8, 9 and 10 of the accompanying drawings. The modified treatment device 101 is identical to the first treatment device 1 other than in that the second container 105 has been modified to comprise a flattened end 106 which lies in a plane perpendicular to the longitudinal axis of the treatment device 101. The flattened end 106 allows the treatment device to be stood vertically upright (i.e. with the longitudinal axis thereof orientated vertically). It will be understood that this is of benefit when the contents of the second container 105 is being applied. In other words, the end 106 allows the second container 105 to be placed on a horizontal surface whilst open, thereby obviating the need for said container 105 to be held in the hand of a user which potentially requires treatment.

## Claims

1. A container assembly (1) comprising first (3) and second containers (5) and a closure element (19) common to the containers, wherein each container (3,5) comprises an opening (7,9) for permitting access to the interior thereof, and wherein the assembly (1) further comprises means for selectively securing each container to the closure element (19) independently of the other container so as to close the opening of the container so secured, the closure element (19) comprising a first applicator (35) associated with the first container (3) and a second applicator (37) associated with the second container (5), wherein each applicator extends through the opening of the associated container when said opening is closed by the closure element (19) and wherein the container assembly (1) has an elongate cylindrical shape; **characterised in that** the container assembly (1) further comprises a planar abrasive area (41) located at one end of the container assembly (1) and arranged so that the longitudinal axis of the container assembly (1) is at an angle to the abrasive area (41).

2. A container assembly (1) according to claim 1, wherein the closure element (19) comprises two applicators (35,37) extending in opposite directions.

3. A container assembly (1) according to claim 2, wherein one of said applicators (35,37) comprises a brush.

4. A container assembly (1) according to claim 2 or 3, wherein one of said applicators (35,37) comprises a wiper.

5. A container assembly (1) according to any of the preceding claims, further comprising vent means.

6. A container assembly (1) according to claim 5, wherein at least one of said containers comprises said vent means.

7. A container assembly (1) according to claim 5 or 6, wherein said vent means comprises a groove (43) cutting across screw threads (21) for securing one of said containers (3,5) to the closure element (19).

8. A container assembly (1) according to any of the preceding claims, wherein the planar abrasive area (41) is provided as an abrasive patch secured to the remainder of the container assembly (1).

9. A container assembly (1) according to claim 8, wherein the abrasive patch is made from carborundum, etched metal, diamond etched metal or emery paper.

10. A method of using the container assembly (1) of any of the preceding claims, comprising the steps of applying, with the associated applicator (35), a first substance stored in said first container (3) to an area of the body of a user; and applying, with the associated applicator (37), a second substance stored in said second container (5) to said area of the body.

11. A method according to claim 10, wherein prior to the application of said first substance, said area of the body is rubbed with the planar abrasive area (41).

12. A method according to claim 10 or 11, wherein the area of the body is a toenail or fingernail.

13. A method according to any of claims 10 to 12, wherein the substance stored in at least one of said containers is a nail coating.

14. A method according to claim 13, wherein said nail coating is a varnish, nail paint or lacquer.

15. A container assembly (1) according to any of claims 1 to 9, wherein at least one container (3,5) comprises a composition for the medical treatment of a user.

16. A container assembly (1) according to claim 15, wherein said composition is for the treatment of a disorder of the fingernail or toenail.

17. A container assembly (1) according to claim 15 or 16, wherein said composition is for the treatment of thickening or hypertrophy of the fingernail or toenail.

18. A container assembly (1) according to any of claims 15 to 17, wherein said composition is for the treatment of a disorder selected from onychauxis and onychogryphosis.

19. A container assembly (1) according to any of claims 15 to 18 wherein said composition comprises a keratolytic agent.

20. A container assembly (1) according to claim 19, wherein said keratolytic agent is selected from urea and a hydroxy-acid.

21. A container assembly (1) according to claim 20, wherein said hydroxy-acid is salicylic acid or lactic acid.

22. A container assembly (1) according to any of claims 15 to 21, wherein said composition comprises a bleaching agent

23. A container assembly (1) according to claim 22, wherein said bleaching agent is hydrogen peroxide.

24. A container assembly (1) according to any of claims 15 to 23, wherein said composition for the medical treatment of a user is a gel.

## Patentansprüche

1. Behälteranordnung (1), die einen ersten (3) und einen zweiten Behälter (5) und ein Verschlusselement (19), das den Behältern gemein ist, umfasst, wobei jeder Behälter (3, 5) eine Öffnung (7, 9) zum Ermöglichen von Zugriff auf dessen Inneres umfasst und wobei die Anordnung (1) weiterhin Mittel zum selektiven Befestigen jedes Behälters an dem Verschlusselement (19) unabhängig von dem anderen Behälter umfasst, um so die Öffnung des so befestigten Behälters zu verschließen, wobei das Verschlusselement (19) einen ersten Applikator (35), der mit dem ersten Behälter (3) verbunden ist, und einen zweiten Applikator (37), der mit dem zweiten Behälter (5) verbunden ist, umfasst, wobei sich jeder Applikator durch die Öffnung des damit verbundenen Behälters erstreckt, wenn die Öffnung von dem Verschlusselement (19) verschlossen ist, und wobei die Behälteranordnung (1) eine längliche zylindrische Form aufweist; **dadurch gekennzeichnet, dass** die Behälteranordnung (1) weiterhin einen ebenen abrasiven Bereich (41) umfasst, der sich an einem Ende der Behälteranordnung (1) befindet und so angeordnet ist, dass der Längszugriff der Behälteranordnung (1) in einem Winkel zu dem abrasiven Bereich (41) ist.

2. Behälteranordnung (1) nach Anspruch 1, wobei das Verschlusselement (19) zwei Applikatoren (35, 37) umfasst, die sich in entgegengesetzte Richtungen erstrecken.

3. Behälteranordnung (1) nach Anspruch 2, wobei einer der Applikatoren (35, 37) eine Bürste umfasst.

4. Behälteranordnung (1) nach Anspruch 2 oder 3, wobei einer der Applikatoren (35,37) einen Wischer umfasst.

5. Behälteranordnung (1) nach einem der vorhergehenden Ansprüche, die weiterhin Entlüftungsmittel umfasst.

6. Behälteranordnung (1) nach Anspruch 5, wobei mindestens einer der Behälter die Entlüftungsmittel umfasst.

7. Behälteranordnung (1) nach Anspruch 5 oder 6, wobei das Entlüftungsmittel eine Nut (43), die über Gewinde (21) verläuft, zum Befestigen eines der Behälter (3,5) an dem Verschlusselement (19) umfasst.

8. Behälteranordnung (1) nach einem der vorhergehenden Ansprüche, wobei der ebene abrasive Bereich (41) als ein abrasives Füllstück bereitgestellt wird, das an dem Rest der Behälteranordnung (1) befestigt ist.

9. Behälteranordnung (1) nach Anspruch 8, wobei das abrasive Füllstück aus Karborund, geätztem Metall, diamantgeätztem Metall oder Sandpapier hergestellt ist.

10. Verfahren zur Verwendung der Behälteranordnung (1) nach einem der vorhergehenden Ansprüche, das die Schritte des Aufbringens einer ersten Substanz, die in dem ersten Behälter (3) aufbewahrt ist, mit dem damit verbundenen Applikator (35) auf einen Bereich des Körpers eines Benutzers; und des Aufbringens einer zweiten Substanz, die in dem zweiten Behälter (5) aufbewahrt ist, mit dem damit verbundenen Applikator (37) auf den Bereich des Körpers umfasst.

11. Verfahren nach Anspruch 10, wobei der Bereich des Körpers vor dem Aufbringen der ersten Substanz mit dem ebenen abrasiven Bereich (41) gerieben wird.

12. Verfahren nach Anspruch 10 oder 11, wobei es sich bei dem Bereich des Körpers um einen Zehennagel oder Fingernagel handelt.

13. Verfahren nach einem der Ansprüche 10 bis 12, wobei es sich bei der Substanz, die in mindestens einem der Behälter aufbewahrt ist, um einen Nagelüberzug handelt.

14. Verfahren nach Anspruch 13, wobei es sich bei dem Nagelüberzug um eine Nagelpolitur, eine Nagelfarbe oder einen Nagellack handelt.

15. Behälteranordnung (1) nach einem der Ansprüche 1 bis 9, wobei mindestens ein Behälter (3, 5) eine Zusammensetzung zur medizinischen Behandlung eines Benutzers umfasst.

16. Behälteranordnung (1) nach Anspruch 15, wobei die Zusammensetzung zur Behandlung einer Erkrankung des Fingernagels oder Zehennagels ist.

17. Behälteranordnung (1) nach Anspruch 15 oder 16, wobei die Zusammensetzung zur Behandlung von Verdickung oder Hypertrophie des Fingernagels oder Zehennagels ist.

18. Behälteranordnung (1) nach einem der Ansprüche 15 bis 17, wobei die Zusammensetzung zur Behandlung einer Erkrankung ist, die aus Onychauxis und Onychogrypose ausgewählt ist.

19. Behälteranordnung (1) nach einem der Ansprüche 15 bis 18, wobei die Zusammensetzung ein keratolytisches Mittel umfasst.

20. Behälteranordnung (1) nach Anspruch 19, wobei das keratolytische Mittel aus Harnstoff und einer Hydroxysäure ausgewählt ist.

21. Behälteranordnung (1) nach Anspruch 20, wobei es sich bei der Hydroxysäure um Salicyclsäure oder Milchsäure handelt.

22. Behälteranordnung (1) nach einem der Ansprüche 15 bis 21, wobei die Zusammensetzung ein Bleichmittel umfasst.

23. Behälteranordnung (1) nach Anspruch 22, wobei es sich bei dem Bleichmittel um Wasserstoffperoxid handelt.

24. Behälteranordnung (1) nach einem der Ansprüche 15 bis 23, wobei es sich bei der Zusammensetzung zur medizinischen Behandlung eines Benutzers um ein Gel handelt.

## Revendications

1. Ensemble de contenants (1) comprenant des premier (3) et second contenants (5) et un élément de fermeture (19) commun aux contenants, dans lequel chaque contenant (3,5) comprend une ouverture (7,9) pour permettre l'accès à l'intérieur de celui-ci, et l'ensemble (1) comprenant en outre un moyen pour fixer sélectivement chaque contenant à l'élément de fermeture (19) indépendamment de l'autre contenant de façon à fermer l'ouverture du contenant ainsi fixé, l'élément de fermeture (19) comprenant un premier applicateur (35) associé au premier contenant (3) et un second applicateur (37) associé au second contenant (5), dans lequel chaque applicateur s'étend à travers l'ouverture du contenant associé quand ladite ouverture est fermée par l'élément de fermeture (19) et dans lequel l'ensemble de contenants (1) a une forme cylindrique allongée ; **caractérisé en ce que** l'ensemble de contenants (1) comprend en outre une zone abrasive plane (41) située à une extrémité de l'ensemble de contenants (1) et agencée de telle sorte que l'accès longitudinal de l'ensemble de contenants (1) soit à un certain angle par rapport à la zone abrasive (41).

2. Ensemble de contenants (1) selon la revendication 1, dans lequel l'élément de fermeture (19) comprend deux applicateurs (35, 37) qui s'étendent dans des sens opposés.

3. Ensemble de contenants (1) selon la revendication 2, dans lequel l'un desdits applicateurs (35, 37) comprend une brosse.

4. Ensemble de contenants (1) selon la revendication 2 ou 3, dans lequel l'un desdits applicateurs (35, 37) comprend un essuyeur.

5. Ensemble de contenants (1) selon l'une quelconque des revendications précédentes, comprenant en outre un moyen d'évent.

6. Ensemble de contenants (1) selon la revendication 5, dans lequel au moins l'un desdits contenants comprend ledit moyen d'évent.

7. Ensemble de contenants (1) selon la revendication 5 ou 6, dans lequel ledit moyen d'évent comprend une rainure (43) qui coupe à travers des filets de vis (21) pour fixer l'un desdits contenants (3,5) à l'élément de fermeture (19).

8. Ensemble de contenants (1) selon l'une quelconque des revendications précédentes, dans lequel la zone abrasive plane (41) est fournie en tant que patin abrasif fixé au reste de l'ensemble de contenants (1).

9. Ensemble de contenants (1) selon la revendication 8, dans lequel le patin abrasif est réalisé en carborundum, métal gravé, métal gravé au diamant ou papier émeri.

10. Procédé d'utilisation de l'ensemble de contenants (1) selon l'une quelconque des revendications précédentes, comprenant les étapes consistant à appliquer, avec l'applicateur associé (35) une première substance stockée dans ledit premier contenant (3) sur une zone du corps d'un utilisateur ; et à appliquer, avec l'applicateur associé (37), une seconde substance stockée dans ledit second contenant (5) sur ladite zone du corps.

11. Procédé selon la revendication 10, dans lequel avant l'application de ladite première substance, ladite zone du corps est frottée avec la zone abrasive plane (41).

12. Procédé selon la revendication 10 ou 11, dans lequel la zone du corps est un ongle d'orteil ou un ongle de doigt.

13. Procédé selon l'une quelconque des revendications 10 à 12, dans lequel la substance stockée dans au moins l'un desdits contenants est un revêtement d'ongle.

14. Procédé selon la revendication 13, dans lequel ledit revêtement d'ongle est un vernis, une peinture ou une laque pour ongles.

15. Ensemble de contenants (1) selon l'une quelconque des revendications 1 à 9, dans lequel au moins un contenant (3, 5) comprend une composition pour le traitement médical d'un utilisateur.

16. Ensemble de contenants (1) selon la revendication 15, dans lequel ladite composition sert au traitement d'une pathologie d'un ongle d'orteil ou d'un ongle de doigt.

17. Ensemble de contenants (1) selon la revendication 15 ou 16, dans lequel ladite composition sert au traitement d'un épaississement ou d'une hypertrophie d'un ongle d'orteil ou d'un ongle de doigt.

18. Ensemble de contenants (1) selon l'une quelconque des revendications 15 à 17, dans lequel ladite composition sert au traitement d'une pathologie sélectionnée parmi l'onychauxis et l'onychogryphose.

19. Ensemble de contenants (1) selon l'une quelconque des revendications 15 à 18, dans lequel ladite composition comprend un agent kératolytique.

20. Ensemble de contenants (1) selon la revendication 19, dans lequel ledit agent kératolytique est sélectionné parmi l'urée et un hydroxy-acide.

21. Ensemble de contenants (1) selon la revendication 20, dans lequel ledit hydroxy-acide est l'acide salicylique ou l'acide lactique.

22. Ensemble de contenants (1) selon l'une quelconque des revendications 15 à 21 dans lequel ladite composition comprend un agent de blanchiment.

23. Ensemble de contenants (1) selon la revendication 22, dans lequel ledit agent de blanchiment est du peroxyde d'hydrogène.

24. Ensemble de contenants (1) selon l'une quelconque des revendications 15 à 23, dans lequel ladite composition du traitement médical d'un utilisateur est un gel.
